# EUROPEAN PATENT APPLICATION

(11) **EP 4 223 308 A1**
(43) Date of publication of application: **09.08.2023**
(21) Application number: 21876028.8
(22) Date of filing: 29.09.2021
(51) Int. Cl.: A61K 38/18, A61P 25/00, A61P 25/18, A23L 33/17, A23L 33/18, A23K 20/147

(54) **COMPOSITION INCLUDING PLGF FOR PREVENTION OR TREATMENT OF NEUROPSYCHOLOGIC DISEASE**

(30) Priority: 29.09.2020 KR 20200127088
(71) Applicant: Cefo Co., Ltd., Seoul 03150 (KR)
(72) Inventor: KIM, Yunhee, Seoul 06280 (KR); LEE, Hyemi, Seoul 02458 (KR); KWON, Hyockman, Suwon-si, Gyeonggi-do 16705 (KR)
(74) Representative: AWA Sweden AB
(86) International application number: PCT/KR2021/013344
(87) International publication number: WO 2022/071753

(57) **Abstract**

The present invention relates to a composition for preventing or treating neuropsychologic diseases, the composition including placental growth factor (PLGF), or a peptide derived therefrom or a fragment thereof. The administration of the PLGF according to the present invention promotes the differentiation of GABAergic (γ-aminobutyric acid, GABA) nerve cells in a memory impairment and autism model to induce nerve regeneration, to enhance memory ability and to ameliorate decline in social cognition, sociability and social sexual preference, whereby a composition for preventing or treating neuropsychologic diseases such as autism, ADHD, mental retardation disorder, and developmental disorder can be provided.

## Description

### [Technical Field]

The present invention relates to a composition for preventing, treating or ameliorating neuropsychologic diseases, comprising placental growth factor (PLGF), a peptide derived therefrom, or a fragment thereof.

### [Background Art]

Neuropsychologic disease refers to a condition in which an imbalance of brain neurotransmitters and genetic, social and environmental factors work in combination to cause abnormalities in thinking, emotion and behavior, and the major examples thereof include depression, anxiety disorder, schizophrenia, dependence, epilepsy, autism spectrum disorder (ASD; autism), attention deficit hyperactivity disorder (ADHD), developmental disorder, and mental retardation disorder. Among them, the autism is known to be caused by genetic mutations in germ cells due to paternal stress, drinking, advanced pregnancy, etc. The current incidence rate has been reported to be 1.5%, but the incidence rate is increasing worldwide, and especially in Korea, there is a report that it has increased to 2.6%. In addition, neuropsychologic diseases such as ADHD and mental retardation disorder are also on the rise, and these diseases are diagnosed early within 3 years from childbirth, so treatment is required at an early stage of onset.

In particular, neuropsychologic diseases including autism, ADHD, schizophrenia, and epilepsy are caused by complex problems of many genes, but in most brain diseases, specific nerve cell death and synapse formation problems, resulting in imbalance of neurotransmitters, lack of inhibitory GABA (γ-aminobutyric acid), regulation of synapse generation, and pruning are attracting attention as the causes. However, although their fundamental treatment is to promote nerve regeneration, proliferation of nerve stem cells, and differentiation of GABAergic nerve cells, few therapeutic agents for such fundamental treatment have been researched and developed yet.

### [Disclosure]

### [Technical Problem]

The present inventors have confirmed that placental growth factor (PLGF), which is one of 16 or more growth factors secreted into nerve stem cells by cerebrovascular cells, has an ameliorating effect on brain diseases such as autism, ADHD, mental retardation and developmental disorders accompanied by memory impairment, thereby completing the present invention.

### [Technical Solution]

One object of the present invention is to provide a pharmaceutical composition for preventing or treating neuropsychologic diseases, the composition comprising, as an active ingredient, (a) placental growth factor (PLGF); or (b) a peptide derived therefrom, or a fragment thereof.

Another object of the present invention is to provide a method for preventing or treating neuropsychologic diseases, comprising administering the composition to an individual.

Still another object of the present invention is to provide a health functional food composition for preventing or ameliorating neuropsychologic diseases, the composition comprising, as an active ingredient, (a) PLGF or (b) a peptide derived therefrom, or a fragment thereof.

Yet another object of the present invention is to provide a feed composition for preventing or ameliorating neuropsychologic diseases, the composition comprising, as an active ingredient, (a) PLGF; or (b) a peptide derived therefrom, or a fragment thereof.

### [Advantageous Effects]

The administration of the placental growth factor (PLGF) or PLGF-derived peptide according to the present invention promotes the differentiation of GABAergic nerve cells in a memory impairment and autism model to induce nerve regeneration, to enhance memory ability and to ameliorate decline in social cognition, sociability and social sexual preference, whereby a composition for preventing or treating neuropsychologic diseases such as autism, ADHD, mental retardation disorder, and developmental disorder can be provided.

### [Description of Drawings]

FIG. 1 shows the results of immunoblotting analysis using NeuN antibody that labels the nucleus of mature neurons, and GAD65/67 (Glutamic acid decarboxylase) and vGAT (vesical GABA transferase) antibodies that label GABAergic nerve cells to determine the optimal dose of placental growth factor (PLGF) to promote neuronal differentiation.
FIG. 2 shows the results analyed by the Western blot method using Sox2, a type 1 neural progenitor cell marker, and DCX antibody, a type 3 neural progenitor cell marker that initiates differentiation while migrating, to determine the optimal dose of PLGF that promotes neural progenitor cell proliferation and early differentiation (neurogenesis).
FIG. 3 shows the results of analyzing cells stained with Ki67 antibody, a proliferating cell marker, and neural progenitor cells stained with nestin antibody, a neural progenitor cell marker, by an immunofluorescence staining method to investigate whether PLGF promotes the proliferation of neural progenitor cells.
FIG. 4 shows the results of analyzing the effect of PLGF on promoting differentiation of GABAergic neural cells by introducing a plasmid into primary cultured neural progenitor cells, wherein the plasmid is obtained by recombining a fluorescent protein, GFP (green fluorescent protein), to a GAD (Glutamic acid decarboxylase) gene promoter, a GABAergic (γ-aminobutyric acid) nerve cell marker.
FIG. 5 is a schematic diagram showing a process of brain transplantation of a human mesenchymal stem cell (hMSC) in which a PLGF expression virus (PLGFF-AAV) is introduced in an animal model of memory impairment and autism.
FIG. 6 shows the results of performing a passive avoidance test (A) and a Morris water maze experiment (B), which measure memory ability in a memory impairment and autism model in which hMSCs into which PLGF-AAV was introduced (PLGF-AAV-hMSC) were transplanted into the hippocampus.
FIG. 7 shows the result of performing a social open field test which measures sociability in a memory impairment and autism model in which PLGF-AAV-hMSC is transplanted into the hippocampus.
FIG. 8 shows the result of a behavioral experiment using a three-room measurement method which measures sociality (session I) in a memory impairment and autism model in which PLGF-AAV-hMSC is transplanted into the hippocampus.
FIG. 9 shows the result of a behavioral experiment using a three-room measurement method which measures social cognition (session II) in a memory impairment and autism model in which PLGF-AAV-hMSC is transplanted into the hippocampus.
FIG. 10 shows the result of a behavioral experiment using a three-room measurement method which measures sexual preference (session III) in a memory impairment and autism model in which PLGF-AAV-hMSC is transplanted into the hippocampus.
FIG. 11 shows the result of staining the death of brain nerve cells through TUNEL staining in the brain tissue of a memory impairment and autism model transplanted with PLGF-AAV-hMSC after a behavioral experiment.
FIG. 12 shows the results of an immunofluorescence staining method which investigate whether or not the proliferation of neural stem cells (sox2+), differentiation into neural cells (NeuN+), and differentiation into oligodendrocyte (CNPase+) are promoted by double staining with BrdU in the brain tissue of a memory impairment and autism model transplanted with PLGF-AAV-hMSC after a behavioral experiment.
FIG. 13 shows the results of whether or not the glutamate neural cell (vGluT 1+) differentiation and the GABAergic neural cell (GAD67+) differentiation are promoted by newly proliferated and BrdU-stained neural progenitor cells in the brain tissue of a memory impairment and autism model transplanted with PLGF-AAV-hMSC after a behavioral experiment.
FIG. 14 is a schematic diagram of a PLGF expression vector (PLGF-AAV).

### [Best Modes of the Invention]

Hereinafter, the present invention will be described in more detail. Meanwhile, descriptions and embodiments disclosed in the present invention may also be applied to other descriptions and embodiments, respectively. That is, all combinations of the various elements disclosed herein fall within the scope of the present invention. In addition, it cannot be said that the scope of the present invention is limited by the specific descriptions described below. In addition, many equivalents to the specific embodiments of the invention described herein can be recognized or ascertained by those skilled in the art using no more than routine experimentation. Also, such equivalents are intended to be encompassed by the present invention.

One aspect of the present invention for achieving the above object is to provide a pharmaceutical composition for preventing or treating neuropsychologic diseases, the composition comprising, as an active ingredient, (a) placental growth factor (PLGF); or (b) a peptide derived therefrom, or a fragment thereof.

As used herein, the term "neuropsychologic disease" refers to a condition in which an imbalance of brain neurotransmitters and genetic, social and environmental factors work in combination to cause abnormalities in thinking, emotion and behavior, and the major examples thereof include depression, anxiety disorder, schizophrenia, dependence, epilepsy, autism spectrum disorder (ASD; autism), attention deficit hyperactivity disorder (ADHD), developmental disorder, and mental retardation disorder, which belong to neuropsychologic disease.

In the present invention, the neuropsychologic disease may be any one or more selected from the group consisting of autism, ADHD, mental retardation disorder and developmental disorder, but is not limited thereto.

In addition, in the present invention, the neuropsychologic disease may be accompanied by memory impairment, but is not limited thereto.

As used herein, the term "placental growth factor (PLGF)" is one of 16 or more growth factors secreted into nerve stem cells by cerebrovascular cells.

The PLGF may exist in three forms of isoform 1, isoform 2 and isoform 3, which are three protein isoforms, and for example, isoform 1 of the PLGF may have the amino acid sequence of SEQ ID NO: 1, isoform 2 may have the amino acid sequence of SEQ ID NO: 2, and isoform 3 may have the amino acid sequence of SEQ ID NO: 3.

As another example, the PLGF may have an amino acid sequence of SEQ ID NO: 1, SEQ ID NO: 2 or SEQ ID NO: 3, consist of an amino acid sequence of SEQ ID NO: 1, SEQ ID NO: 2 or SEQ ID NO: 3, or include an amino acid sequence set forth in SEQ ID NO: 1, SEQ ID NO: 2 or SEQ ID NO: 3, but is not limited thereto. The sequence of SEQ ID NO: 1, SEQ ID NO: 2 or SEQ ID NO: 3 can be confirmed in NCBI Genbank, a known database.

Specifically, the PLGF may have SEQ ID NO: 1, SEQ ID NO: 2 or SEQ ID NO: 3, and/or an amino acid sequence having at least 70% homology or identity with SEQ ID NO: 1, SEQ ID NO: 2 or SEQ ID NO: 3. In addition, as long as it is an amino acid sequence having such homology or identity and exhibiting a function corresponding to the PLGF, it is apparent that a PLGF having an amino acid sequence in which a part of the sequence is deleted, modified, substituted or added is also included within the scope of the present invention.

That is, although it is described as 'a protein or polypeptide including an amino acid sequence described in a specific sequence number' or 'a protein or polypeptide consisting of an amino acid sequence described in a specific sequence number' in the present application, it is obvious that a protein consisting of an amino acid sequence in which a part of the sequence is deleted, modified, substituted or added may also be used in the present invention, provided that it has an activity identical to or corresponding to that of a polypeptide consisting of the amino acid sequence of that SEQ ID NO. For example, it is apparent that a 'polypeptide comprising an amino acid sequence of SEQ ID NO: 1' may belong to a 'polypeptide comprising an amino acid sequence of SEQ ID NO: 1' if it has the same or corresponding activity.

As used herein, the term "PLGF-derived peptide" may include any peptide selected from peptides constituting PLGF without limitation, and specifically, the PLGF-derived peptide may include two or more consecutive amino acids, and may also include a fragment thereof. For example, the PLGF-derived peptide may include two or more consecutive amino acids derived from isoform 1, isoform 2 or isoform 3, or may be a fragment thereof, but is not limited thereto.

In the present invention, (a) PLGF or (b) a PLGF-derived peptide or fragment thereof may be included in the composition in any one form selected from the group consisting of a polypeptide sequence of PLGF or a fragment thereof; a polynucleotide sequence encoding the polypeptide sequence or a fragment thereof; and a vector comprising the polynucleotide sequence, but is not limited thereto.

In the present invention, a gene encoding the PLGF may include a base sequence encoding an amino acid sequence of SEQ ID NO: 1, SEQ ID NO: 2 or SEQ ID NO: 3 and more specifically, may include, have, or consist of an base sequence of SEQ ID NO: 4, SEQ ID NO: 5 or SEQ ID NO: 6, but is not limited thereto. The base sequence of SEQ ID NO: 4, SEQ ID NO: 5 or SEQ ID NO: 6 may be obtained from GenBank, a known database.

As used herein, the term "polynucleotide" refers to a DNA or RNA strand having a certain length or longer, which is a polymer of nucleotides in which nucleotide monomers are covalently linked in a long chain shape, and more specifically, a polynucleotide fragment encoding the variant. The polynucleotide may be described as a gene if it is an aggregate of polynucleotides capable of functioning. In the present invention, the polynucleotide and the gene may be used interchangeably.

Specifically, the polynucleotide of the present invention may be subjected to various modifications in the coding region within a range that does not change the amino acid sequence of the polypeptide due to codon degeneracy or in view of codons preferred in an organism intended to express the polypeptide. Specifically, any polynucleotide sequence encoding PLGF consisting of the amino acid sequence of SEQ ID NO: 1 may be included without limitation.

In addition, if it is a probe that can be prepared from a known gene sequence, for example, a sequence encoding the PLGF consisting of the amino acid sequence of SEQ ID NO: 1, SEQ ID NO: 2 or SEQ ID NO: 3 by hybridization with a sequence complementary to all or part of the base sequence under a stringent condition, it may be included without limitation. The term "stringent condition" means a condition that enables specific hybridization between the polynucleotides. Such a condition is well known in the art. For example, there can be exemplified a condition in which genes with high homology or identity, specifically genes having homology or identity of 40% or more, specifically 90% or more, more specifically 95% or more, even more specifically 97% or more, particularly specifically 99% or more hybridize to each other, and genes with lower homology or identity do not hybridize; or a condition of washing once, specifically 2 to 3 times at salt concentration and temperature equivalent to 60°C, 1YSSC, 0.1% SDS, specifically 60°C, 0.1YSSC, 0.1% SDS, more specifically 68°C, 0. 1YSSC, 0.1% SDS, which are washing conditions for normal southern hybridization.

Hybridization requires that two nucleic acids have complementary sequences, although mismatches between bases are possible depending on the stringency of hybridization. The term "complementary" is used to describe the relationship between nucleotide bases capable of hybridizing to each other. For example, for DNA, adenosine is complementary to thymine, and cytosine is complementary to guanine. Accordingly, the present invention may also include isolated nucleic acid fragments complementary to the overall sequence as well as substantially similar nucleic acid sequences.

Specifically, polynucleotides having homology or identity can be detected using hybridization conditions including a hybridization step at a Tm value of 55°C and using the above-described conditions. In addition, the Tm value may be 60°C, 63°C, or 65°C, but is not limited thereto, and may be appropriately adjusted depending on the purpose by those skilled in the art.

The appropriate stringency for hybridizing polynucleotides depends on the length of the polynucleotides and the degree of complementarity, and the parameters are well known in the art (see Sambrook et al., supra, 9.50-9.51, 11.7-11.8).

As used herein, the terms "homology" and "identity" refer to a degree to which two given amino acid sequences or base sequences are related, and may be expressed as a percentage. The terms "homology" and "identity" can often be used interchangeably.

Sequence homology or identity of a conserved polynucleotide or polypeptide is determined by a standard arrangement algorithm, and a default gap penalty established by the program used may be used together. Substantially, homologous or identical sequences may generally hybridize to at least about 50%, 60%, 70%, 80%, or 90% or more of the total or full-length of the sequence under moderate or high stringent conditions. Hybridization also considers polynucleotides containing degenerate codons instead of codons in polynucleotides.

The homology or identity of the polypeptide or polynucleotide sequence can be determined, for example, by an algorithm BLAST according to the literature [see: Karlin and Altschul, Pro. Natl. Acad. Sci. USA, 90, 5873(1993)], or FASTA by Pearson (see: Methods Enzymol., 183, 63, 1990). Based on such an algorithm BLAST, a program called BLASTN or BLASTX has been developed (see http://www.ncbi.nlm.nih.gov). In addition, whether or not any amino acid or polynucleotide sequence has homology, similarity or identity can be confirmed by comparing the sequences by Southern hybridization experiments under defined stringent conditions, and the defined appropriate hybridization conditions are within the scope of the art, and can be determined by methods well known to those skilled in the art (e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology).

As used herein, the term "vector" refers to a DNA product containing a polynucleotide sequence encoding a desired protein in a suitable host in a form operably linked to a regulatory sequence suitable for expressing the target protein. The expression regulatory sequence may include a promoter capable of initiating transcription, an operator sequence for regulating such transcription, a sequence encoding a suitable mRNA ribosome binding site, and a sequence regulating the termination of transcription and translation. After transformation into an appropriate host cell, the vector can replicate or function independently of the host genome, and can be integrated into the genome itself.

The vector used in the present invention is not particularly limited as long as it can be used for gene therapy while replicating in a host cell, and any vector known in the art may be used. For example, examples of vectors commonly used for gene therapy include viral vectors or non-viral vectors in a natural or recombinant state. As specific examples, the viral vector may include an adenovirus, a retrovirus, a lentivirus, an adeno-associated virus (AAV), an oncolytic virus, and a herpes simplex virus vector and the like, and non-viral vectors may include a plasmid and a liposome. Specifically, the vector usable in the present invention may be an AAV vector as a viral vector for gene therapy, but is not particularly limited thereto, and any known viral vector for gene therapy may be used.

Since the viral vector for gene therapy is infected only once during viral infection and does not proliferate the virus, it does not infect surrounding cells and is safely used for human treatment. Among them, AAV is recognized as the most stable type of vector when used clinically for human treatment, and is currently the most used vector in clinical gene therapy worldwide. The present invention may also include a viral vector in the form of a peptide, and may further include a viral vector in the form of a PLGF-derived peptide containing a portion of the PLGF sequence or various DNA vectors including the viral vector without limitation.

As used herein, the term "transformation" means that a recombinant vector containing a polynucleotide encoding a target protein is introduced into a host cell, and the protein encoded by the polynucleotide is expressed in the host cell.

In addition, the term "operably linked" as used herein means that the polynucleotide sequence is functionally linked to a promoter sequence or an expression regulatory region that initiates and mediates transcription of a polynucleotide encoding a target protein of the present invention. The operable linkage can be produced using a genetic recombination technique known in the art, and site-specific DNA cleavage and linkage can be made using cleaving and linking enzymes known in the art, but is not limited thereto.

In the present invention, the "target protein" may be PLGF or a peptide derived therefrom or a fragment thereof.

The PLGF, PLGF-derived peptide or fragment thereof according to the present invention may be included in the pharmaceutical composition in the form of: a polypeptide sequence of PLGF or a fragment thereof; a polynucleotide sequence encoding the polypeptide sequence or a fragment thereof; or a vector comprising the polynucleotide sequence, thereby increasing the level of PLGF in an individual to which the composition is administered and thus exhibiting an effect of preventing or treating neuropsychologic diseases.

As used herein, the term "prevention" refers to any action that suppresses or delays the onset of the neuropsychologic diseases by administration of the composition.

As used herein, the term "treatment" refers to any action that ameliorates or beneficially changes the symptoms of the neuropsychologic diseases by administration of the composition.

The pharmaceutical composition comprising the PLGF, PLGF-derived peptide or fragment thereof as an active ingredient according to the present invention may further include an appropriate carrier, excipient or diluent commonly used in the preparation of the pharmaceutical composition. In this case, the content of the active ingredient included in the composition is not particularly limited, but may include 0.0001 wt% to 10 wt%, preferably 0.001 wt% to 1 wt%, based on the total weight of the composition.

The pharmaceutical composition may have any one dosage form selected from the group consisting of: tablets, pills, powders, granules, capsules, suspensions, liquid for internal use, emulsions, syrups, sterile aqueous solutions, non-aqueous solutions, suspensions, emulsions, freeze-dried preparations and suppositories, and may be several oral or parenteral dosage forms. When formulated, it is prepared using commonly used diluents or excipients such as fillers, extenders, binders, wetting agents, disintegrants, and surfactants. Solid formulations for oral administration include tablets, pills, powders, granules, capsules, etc., and these solid preparations are prepared by mixing one or more compounds with at least one excipient, such as starch, calcium carbonate, sucrose or lactose, gelatin, etc. In addition to simple excipients, lubricants such as magnesium stearate and talc are also used. Liquid formulations for oral administration include suspensions, internal solutions, emulsions, syrups, etc, which may contain various excipients such as wetting agents, sweeteners, fragrances, and preservatives in addition to commonly used simple diluents such as water and liquid paraffin. Formulations for parenteral administration include sterile aqueous solutions, non-aqueous solutions, suspensions, emulsions, freeze-dried preparations, and suppositories. As non-aqueous solvents and suspending agents, propylene glycol, polyethylene glycol, vegetable oils such as olive oil, and injectable esters such as ethyl oleate may be used. As the base of the suppository, witepsol, macrogol, tween 61, cacao butter, laurin butter, glycerogelatin, and the like may be used.

The composition of the present invention may be administered in a pharmaceutically effective amount.

As used herein, the term "pharmaceutically effective amount" means an amount sufficient to treat a disease with a reasonable benefit/risk ratio applicable to medical treatment, and the effective dose level can be determined according to factors, including individual type and severity, age, sex, disease type, drug activity, drug sensitivity, administration time, administration route and discharge rate, treatment period, drugs used concurrently, and other factors well known in the medical field. The composition of the present invention may be administered individually or in combination with other therapeutic agents, and may be administered sequentially or simultaneously with conventional therapeutic agents. It may also be administered singly or multiply. In consideration of all of the above factors, it is important to administer an amount that can obtain the maximum effect with a minimum amount without side effects, and such amount can be easily determined by those skilled in the art. The preferred dosage of the composition of the present invention varies depending on the patient's condition and weight, degree of disease, drug form, administration route and period, but for the desired effect, it is desirable that the pharmaceutical composition of this invention is administered at 0.0001 to 100 mg/kg, preferably 0.001 to 10 mg/kg per day. Administration may be made once a day, or divided several times. The composition may be administered to various mammals such as rats, livestock and humans through various routes, and may be administered without limitation by any conventional methods known in the art, for example, by oral, rectal or intravenous, intramuscular, subcutaneous, intrauterine intrathecal or intracerebrovascular injection.

In addition, the pharmaceutical composition of the present invention may also be used in the form of veterinary medicines as well as medicines applied to humans.

The PLGF, PLGF-derived peptide or fragment thereof according to the present invention can promote the regeneration of nerve cells in the hippocampus.

The PLGF, PLGF-derived peptide or fragment thereof according to the present invention can promote the differentiation of nerve cells.

Specifically, the PLGF, PLGF-derived peptide or fragment thereof according to the present invention can promote the regeneration of nerve cells by promoting the differentiation of GABAergic (γ-aminobutyric acid) nerve cells.

Diseases such as attention deficit/hyperactivity disorder, stroke, schizophrenia, and autism are known to be caused by an imbalance between excitatory cells and GABAergic nerve cells. In order to ameliorate these diseases, it is necessary to promote the production or differentiation of GABA, an inhibitory signaling substance, rather than an excitatory signaling substance with a complex signal transduction system. Among the genes involved in the differentiation into GABAergic nerve cells, the GAD (glutamic acid decarboxylase) gene is an enzyme that catalyzes the decarboxylation of glutamate, and is known to synthesize the GABA, an inhibitory neurotransmitter, through this process.

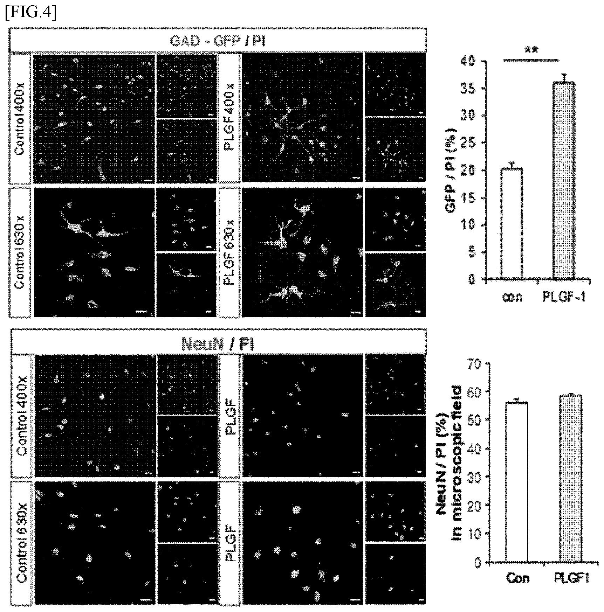

Accordingly, in an embodiment of the present invention, it was confirmed whether the PLGF promotes differentiation into GABAergic nerve cells in the course of neural cell differentiation of primary neural progenitor cells. As a result, NeuN-stained cells did not increase when treated with the PLGF at a concentration of 30 ng/ml during the primary neural progenitor cell differentiation process, but GFP-positive cells increased compared to the Con group, whereby the PLGF promoted the differentiation into GABAergic nerve cells in the process of neural cell differentiation (FIG. 4).

The PLGF, PLGF-derived peptide or fragment thereof according to the present invention can improve memory.

In the present invention, a model of memory impairment and autism induced by ibotenic acid (IBO) was prepared.

The IBO is a compound that damages neurons of striatum, hippocampus formation, substantia nigra, and piriform cortex when injected into the brain of a rat. In specific experiments, by administering it to be injected into three areas in the entorhinal cordex, nerve damage to the dorsal and ventral sides of the hippocampus can be induced, leading to the memory damage and autism (Miyuki Sadamatsu, Congenital Anomalies 2006; 46, 1-9, Review of animal models for autism: implication of thyroid hormone).

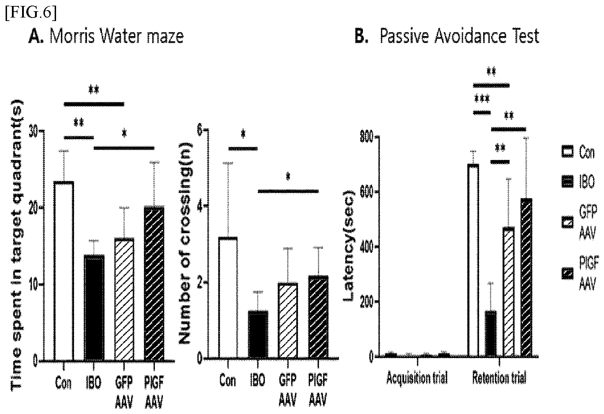

Accordingly, in one embodiment of the present invention, memory ability was measured for the memory impairment and autism model administered with IBO through a passive avoidance test and a Morris water maze test, and all results indicated that the memory was enhanced (FIG. 6A-B).

The PLGF, PLGF-derived peptide or fragment thereof of the present invention can improve sociability or social cognitive ability.

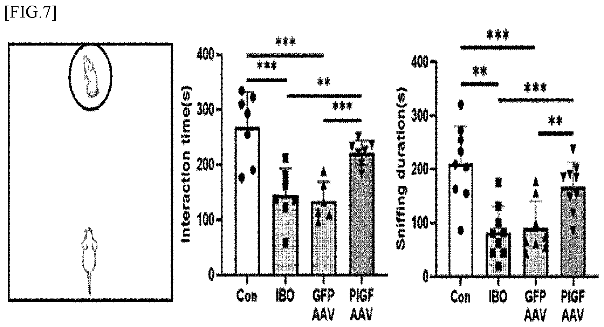

In one embodiment of the present invention, it was confirmed whether or not social behavior, social cognition, sociability, and social sexual preference were restored by PLGF administration through a behavioral experiment for the memory impairment and autism model administered with IBO, and as a result, social behavior, social cognition, sociability and social sexual preference were restored in the PLGF-administered group compared to the IBO group or the positive control group (FIGS. 7-10).

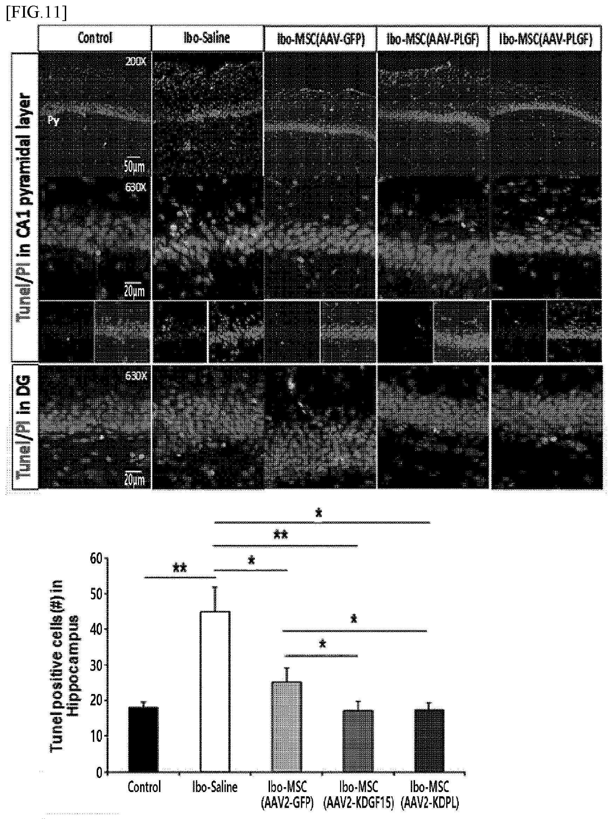

In another embodiment of the present invention, brain tissues of the memory impairment and autism model after the behavioral experiment were analyzed, and as a result, cell death was significantly increased in the IBO-administered group, but the cell death was significantly suppressed in the PLGF-AAV-hMSC-administered group compared to the IBO-administered group and the GFP-AAV-hMSC-administered group, whereby the PLGF inhibited death of brain nerve cells and increased survival in the memory impairment and autism model (FIG. 11).

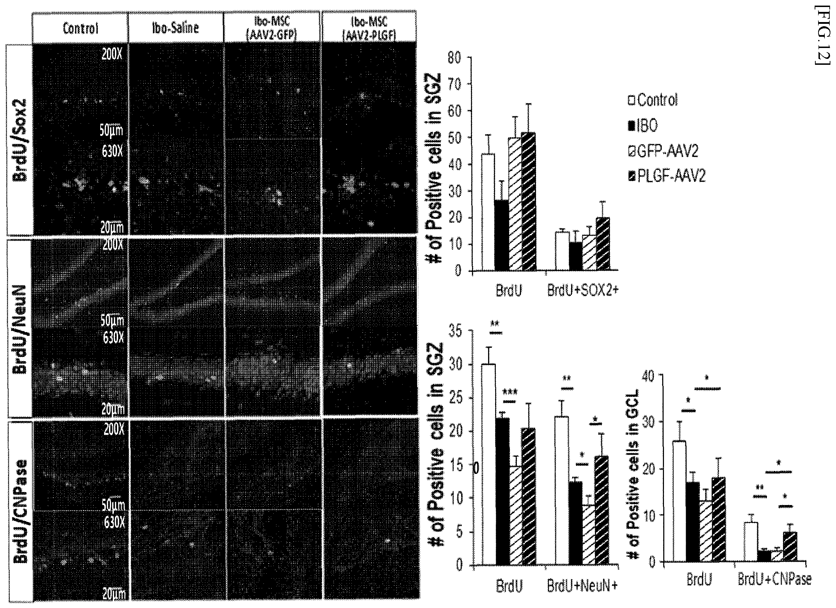

In addition, in another embodiment of the present invention, when the brain tissue of the memory impairment and autism model after the behavioral experiment was labeled with cells that were proliferating at that time using BrdU, the number of SOX2, a marker of neural stem cells, tended to increase in the PLGF-AAV-administered group. Afterwards, at the time of the differentiation of neural cells, the number of NeuN, a marker of mature neural cells, increased significantly in the PLGF-AAV-administered group, and the number of oligodendrocytes (CNPase) also increased compared to the IBO-administered group or the GFP-AAV-administered group. Glutamate neural cells (vGluT1) and GABAergic neural cells (GAD) were also increased compared to the IBO-administered group and the GFP-AAV-administered group. In addition, the increase in these GABAergic neural cells was also increased in the hippocampal CA region (Torcato Meira et al., Nature communications, 2018), which is known to be associated with sociability (FIGS. 12-13 ).

As such, the present invention is meaningful in revealing for the first time that the PLGF promotes the differentiation of GABAergic nerve cells in memory impairment and autism models to induce nerve regeneration, enhance memory ability, and ameliorate decline in social cognition, sociability and social sexual preference, thereby exerting the effect of preventing or treating neuropsychologic diseases, especially autism, ADHD, mental retardation disorder, and developmental disorder.

In addition, a peptide derived from the PLGF or a fragment thereof can also exhibit the same effect as the PLGF as described above.

Another aspect of the present invention is to provide a method for preventing or treating neuropsychologic diseases, comprising administering the pharmaceutical composition of the present invention to an individual.

The terms used herein are as described above.

As used herein, the term "individual" refers to any animal, excluding humans, in which neuropsychologic diseases have occurred or may occur. By administering the pharmaceutical composition of the present invention to an individual suspected of having the neuropsychologic disease, the individual can be effectively treated.

As used herein, the term "administration" means the pharmaceutical composition of the present invention is introduced into an individual suspected of having a neuropsychologic disease by any suitable method, wherein the administration can be performed through various routes such as oral or parenteral, as long as it can reach the target tissue.

The pharmaceutical composition of the present invention may be administered in a pharmaceutically effective amount, as described above.

The pharmaceutical composition of the present invention may be applied to any individual for the purpose of preventing or treating neuropsychologic diseases without particular limitation. For example, it may be applied to non-human animals such as monkeys, dogs, cats, rabbits, guinea pigs, rats, mice, cattle, sheep, pigs, goats, etc., birds and fish, and the pharmaceutical composition may be administered parenterally, subcutaneously, intraperitoneally, intrapulmonaryly, or intranasally, and if necessary, may be administered by any suitable method including intralesional administration for local treatment. The preferred dosage of the pharmaceutical composition of the present invention varies depending on the condition and weight of the individual, the degree of disease, the drug form, the route and period of administration, and can be appropriately selected by those skilled in the art. For example, the composition may be administered by oral, rectal or intravenous, intramuscular, subcutaneous, intrauterine intrathecal or intracerebrovascular injection, but is not limited thereto.

Still another aspect of the present invention is to provide a health functional food composition for preventing or ameliorating neuropsychologic diseases, the composition comprising, as an active ingredient, (a) PLGF; or (b) a peptide derived therefrom, or a fragment thereof.

The terms used herein are as described above.

As used herein, the term "amelioration" refers to any action that ameliorates or benefits the symptoms of an individual suspected or diagnosed with a neuropsychologic disease by using the composition.

The food composition of the present invention may include a food-acceptable salt, and as such a salt, an acid addition salt formed by a food-acceptable free acid or a metal salt formed by a base is useful. For example, an inorganic acid and an organic acid may be used as the free acid. Hydrochloric acid, sulfuric acid, hydrobromic acid, sulfurous acid, phosphoric acid, or the like may be used as the inorganic acid, and citric acid, acetic acid, maleic acid, fumaric acid, gluconic acid, methanesulfonic acid, or the like may be used as the organic acid. In addition, an alkali metal salt or an alkaline earth metal salt, sodium salt, potassium salt or calcium salt may be used as the metal salt. However, it is not limited thereto.

The food composition of the present invention includes the form of pills, powders, granules, needles, tablets, capsules, liquids, or the like, and the food to which the composition may be added includes various foods, such as beverages, chewing gum, tea, vitamin complexes, and health supplements.

In addition to the active ingredient contained as an essential ingredient, the food composition of the present invention may include other ingredients without particular limitation, and may contain various herbal extracts, food supplement additives, natural carbohydrates, or the like as additional ingredients like conventional foods. The content of the active ingredient in the food composition may be suitably determined according to the purpose of use (prevention, amelioration or therapeutic treatment). In this case, the content of the active ingredient included in the composition is not particularly limited, but may include 0.0001 wt% to 10 wt%, preferably 0.001 wt% to 1 wt%, based on the total weight of the composition.

In addition, the food supplement additive may include food supplement additives commonly used in the art, for example, a flavoring agent, a sweetener, a coloring agent, a filler, a stabilizer, and the like.

Examples of the natural carbohydrate include monosaccharides such as glucose, fructose and the like; disaccharides such as maltose, sucrose and the like; and polysaccharides, for example, conventional sugars such as dextrin and cyclodextrin, and sugar alcohols such as xylitol, sorbitol, and erythritol. As the flavoring agent other than those described above, natural flavoring agents (e.g., rebaudioside A, glycyrrhizin, etc.) and synthetic flavoring agents (saccharin, aspartame, etc.) may be advantageously used.

In addition to the above, the food composition of the present invention may include various nutrients, vitamins, minerals (electrolytes), flavoring agents such as synthetic and natural flavoring agents, coloring agents and fillers (cheese, chocolate, etc.), pectic acid and its salts, alginic acid and its salts, organic acids, protective colloidal thickeners, pH adjusters, stabilizers, preservatives, glycerin, alcohols, carbonating agents used in carbonated beverages, and the like. In addition, it may contain the pulp for the production of natural fruit juice and fruit juice beverages and vegetable beverages. These components may be used alone or in combination.

In the present invention, the health supplement may include a health functional food and a health food.

The health functional food is the same term as food for special health use (FoSHU), and refers to a food with high medicinal and medical effects processed so that the bioregulatory function is efficiently exhibited in addition to nutritional supply. Here, the "function(al)" means having a useful effect for health use, such as nutrient regulation or physiological action for the structure and function of the human body.

Food comprising the food composition of the present invention can be prepared by a method commonly used in the art, and when prepared, raw materials and components commonly used in the art may be added. In addition, the food may also be prepared in any formulation without limitation as long as it is a formulation recognized as a food. The food composition of the present invention may be prepared in various forms of formulations; and unlike general drugs, since it uses food as a raw material, it has the advantage of not having side effects that may occur when taking drugs for a long time, and can be excellent in portability.

Yet another aspect of the present invention is to provide a feed composition for preventing or ameliorating neuropsychologic diseases, the composition comprising, as an active ingredient, (a) PLGF; or (b) a peptide derived therefrom, or a fragment thereof.

The terms used herein are as described above.

As used herein, the term "feed" refers to any natural or artificial diet, one-meal, etc., or a component of the one-meal, which are intended to be eaten, ingested and digested by an animal, or suitable therefor.

The type of the feed is not particularly limited, and any feed commonly used in the art may be used. Non-limiting examples of the feed include plant feeds such as grains, root fruits, food processing by-products, algae, fibers, pharmaceutical by-products, oils and fats, starches, gourds or grain by-products; and animal feeds such as proteins, inorganic materials, oils and fats, minerals, oils and fats, single cell proteins, zooplankton, or food. These may be used alone or in mixture of two or more, but is not limited thereto.

The feed composition of the present invention may be applied to the diet of many animals including mammals, poultry, fish and shellfish, that is, feed. Specifically, it may be used for commercially important mammals such as pigs, cattle, and goats, zoo animals such as elephants and camels, and livestock such as dogs and cats. Poultry of commercial importance may include chickens, ducks and geese, and may include commercially raised fish and crustaceans such as trout and shrimp.

In addition to the PLGF, PLGF-derived peptide or fragments thereof, the feed composition of the present invention may be further mixed for administration with one or more of: organic acids such as citric acid, fumaric acid, adipic acid, and lactic acid; phosphates such as potassium phosphate, sodium phosphate, and polymerized phosphate; natural antioxidants such as polyphenols, catechins, tocopherols, vitamin C, green tea extract, chitosan, and tannic acid. In addition, diluents, dispersants, surfactants, binders, or lubricants may be additionally added to prepare formulations for injections such as aqueous solutions, suspensions, emulsions, etc., capsules, granules, or tablets.

In addition, the feed composition of the present invention may be used together with various supplements such as amino acids, inorganic salts, vitamins, antioxidants, antifungal agents, and antibacterial agents as auxiliary ingredients, vegetable protein feed such as ground or crushed wheat, barley, and corn, animal protein feed such as blood meal, meat meal, and fish meal, nutritional supplements, growth promoters, digestion and absorption promoters, and disease preventives in addition to the main ingredients such as animal and vegetable fats.

The feed composition may include a feed additive. The feed additive of the present invention corresponds to an auxiliary feed under the Feed Management Act.

When the feed composition of the present invention is used as a feed additive, the feed composition may be added as it is or be used together with other ingredients, and may be appropriately used according to a conventional method. The dosage form of the feed composition may be prepared as an immediate-release or sustained-release formulation in combination with a non-toxic pharmaceutically acceptable carrier. Such edible carriers may be corn starch, lactose, sucrose, propylene glycol. In the case of a solid carrier, it may be in dosage forms such as tablets, powders, and troches; and in the case of a liquid carrier, it may be in dosage forms such as syrups, liquid suspensions, emulsions, and solutions. In addition, the administration agent may contain preservatives, lubricants, solution accelerators, stabilizers, and may contain substances useful for the prevention, treatment or amelioration of other diseases.

### EXAMPLES

Hereinafter, the present invention will be described in more detail by way of examples. However, the following examples are only for illustrating the present invention, and the scope of the present invention is not limited thereto.

### Example 1. Investigation of optimal dose of placental growth factor (PLGF) in the process of nerve cell differentiation

To obtain the optimal dose of PLGF that promotes the expression of differentiation markers in neural progenitor cells, immunoblotting experiments were performed using NeuN antibody that labels the nuclei of mature neurons, glutamic acid decarboxylase (GAD65/67) and vesicular GABA transferase (vGAT) that label GABAergic nerve cells.

Specifically, hippocampal-derived neural stem cells were isolated from the brain of E16 rat embryos. Hippocampal ridges were minced from the embryonic forebrains of E16 rats using sterile fine tweezers under a dissecting microscope. The tissues were collected, mechanically separated using a pipette in Ca²⁺/Mg²⁺-freeHBSS (Invitrogen) solution, and planted at 19,000 cells/cm² in 35 mm culture dishes precoated with 15 µg/ml poly-L-ornithine and 1 µg/ml fibronectin (Sigma). The cells were cultured under the condition of 5% CO₂ for 3 days using a serum-free N2 medium (medium for proliferation) supplemented with 10 ng/mL basic fibroblast growth factor(bFGF, Invitrogen). Thereafter, the cells were removed using 0.05% trypsin/EDTA (ethylene-diamine-tetraacetic acid), and neural progenitor cells were planted on a 12 mm glass coverglass (Belco) for immunostaining and on a 100 mm dish for immunoblotting and cultured in N2 medium for proliferation containing bFGF for 1 day. Thereafter, in the proliferation condition, the medium continuously containing bFGF was treated with PLGF at the indicated concentration and cultured for 2-3 days; and in the differentiation condition, PLGF was added to the medium for differentiation not containing bFGF, which was cultured for 3-7 days. In the control group (vehicle), physiological saline was added in the same volume instead of PLGF.

After 3-7 days of differentiation of the cultured neural stem cells, the medium was removed and washed once with PBS (Phosphate-buffered saline). Then, all the cells were lysed using a lysis buffer to which a protease inhibitor was added. The lysate was loaded in a 10% gel in the same volume, then transferred to a PVDF (Polyvinylidene Fluoride) membrane and blocked with 2% BSA in TBST or 5% skim milk in TBST at room temperature for 1 hour. As primary antibodies, rb-GAD65/67 (1: 1000), mo-NeuN (1: 1000), mo-Vgat (1:700), and mo-actin (1:2000) were used, and the primary antibodies were diluted with 2% BSA in TBST, reacted at 4°C for 16 hours, and then washed with 1X TBST. As secondary antibodies, mo-HRP (1:5000) and rb-HRP (1:5000) were used, and the secondary antibodies were diluted in 2% skim milk in TBST or 2% BSA in TBST, reacted for 1 hour, and then developed on films. As control antibodies, mo-tubulin (Millipore, 05-661, 1:2000) and mo-actin (Santa cruz, SC-47778, 1:2000) were used.

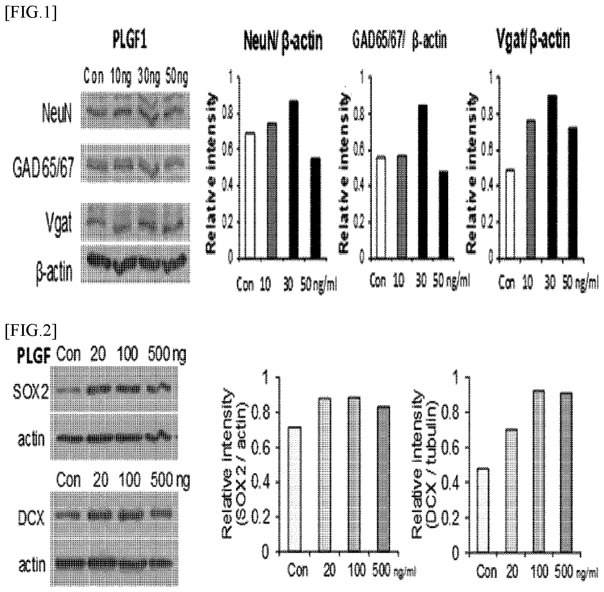

As a result, when treated with PLGF at 10-50 ng/ml, the NeuN did not significantly increase, but the amount of GAD65/67 and vGAT protein increased significantly, and the NeuN, GAD65/67 and vGAT protein all increased the most at 30 ng/ml concentrations (FIG. 1).

Next, for proliferating neural progenitor cells, Sox2 (mo-Sox2: R&D system, MAB2018, 1:1000), a Type 1 neural progenitor cell marker, and DCX (goat-DCX: Santa cruz, SC-8066, 1:1000)), a migrating neural progenitor cell marker that initiates Type 3 differentiation, were immunoblotted, and as a result, when the PLGF was added at 20-500 ng/ml, all showed a tendency to increase (FIG. 2). Specifically, both Sox2 and DCX were significantly increased at 20-100 ng/ml.

Therefore, in the following Examples, experiments were conducted using a concentration of 30 - 100 ng/ml, which is the optimal dose of PLGF derived from the above experimental results.

### Example 2. Investigation of the effects of PLGF on promoting neural cell proliferation and differentiation

To investigate whether or not PLGF promotes proliferation of neural progenitor cells, the primary cultured neural progenitor cells are treated with PLGF, and then proliferating cells were fluorescently stained with Ki67 antibody, and neural progenitor cells with Nestin antibody.

Specifically, the neural progenitor cells were cultured in the same manner as in Example 1, and after removing all the mediums, they were washed once with PBS. After being fixed for 15 minutes with 4% PFA (paraformaldehyde) in PBS, they were washed twice with PBS again. After washing the cover slip to which the cells were attached with PBST, 0.5% Triton X-100-PBST was added thereto and reacted for 10 minutes. Again, blocking was performed with 2% BSA in PBST or 5% normal serum (Normal donkey serum: Jackson lab, 017-000-121, Normal horse serum: Sigma, H0146); and as primary antibodies, rb-Ki67(Abcam, AB15580, 1:500), mo-NeuN(Millipore, MAB377B, 1:500), mo-Tuj1(Sigma, T8660, 1:3000), mo-Flag(Sigma, F1804, 1:500), and mo-Nestin(Millipore, MAB353, 1:200) were added to 2% BSA in PBS and reacted overnight at 4°C. The next day, they were washed with PBST, and then, as secondary antibodies, mo-Alexa 488(Invitrogen, A21202, 1:700), mo-Cy3(Jackson lab, 715-165-151, 1:500), and rb-Alexa 488(Jackson lab, 711-546-152, 1:700) were added to PBST and reacted at room temperature for 1 hour. Cell nuclei were stained with DAPI (1 µg/mL, Sigma), mounted on slides, scanned with a confocal laser microscope, and photographed. Heo SR et al.(2009) and Han AM t al.(2012) were referred to in the above method.

In the case of cell count and data analysis, the number of cell nuclei labeled with DAPI and the number of cells stained with each differentiation marker were counted, respectively, and the number of each differentiation marker was expressed as a percentage of the number of cell nuclei. In this case, the number of cells that matched exactly in shape and location and had the shape of a nerve cell was counted. For example, in NeuN cell count measurement, the number of cells with a round shape in a nucleus and overlapping staining with DAPI was counted, and non-specific signals appearing at the edge of the slide were not included. In data analysis, the control group and the experimental group were compared through one-way analysis of variance (ANOVA). The statistical significance of the data was set as p<0.05.

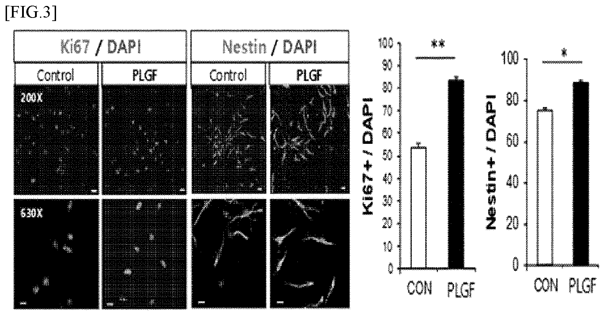

As a result, Ki67-stained proliferating cells and nestin-stained neural progenitor cells were significantly increased compared to the control group (FIG. 3).

### Example 3. Investigation of the effects of PLGF on promoting GABAergic (γ-aminobutyric acid) nerve cell differentiation

In order to efficiently investigate the differentiation of the primary neural progenitor cells into GABAergic nerve cells by PLGF, a plasmid (GAD-GFP) capable of easily and effectively observing and detecting the differentiation into the GABAergic nerve cells with the naked eye by recombining green fluorescent protein (GFP), a fluorescent protein, into the promoter area of the human GAD gene was used. When the activity of the promoter is increased, the differentiation into GABAergic nerve cells is promoted, and whether or not the activity of the promoter is increased can be monitored by the expression level of a reporter gene operably linked thereto. Accordingly, it can be seen that the differentiation into GABAergic nerve cells is promoted when the expression level of the reporter gene is increased. In addition, it is possible to easily observe the differentiation of GABAergic nerve cells using a transcription factor that binds to the promoter.

Specifically, on day 3 after culturing the primary neural progenitor cells, the cells were separated from the dish with 0.05% trypsin and subcultured, and then all groups were transfected with GAD-GFP DNA. The cells were seeded in a 24-well plate and cultured in N2 medium containing 10 ng/mL bFGF for 24 hours. N2 medium for differentiation without bFGF was treated with PLGF at a concentration of 30 ng/ml, and after 3 days, it was fixed and stained.

As a result, NeuN-stained cells did not increase when treated with the PLGF at a concentration of 30 ng/ml, but GFP-positive cells increased compared to the Con group, whereby the PLGF promoted the differentiation into GABAergic nerve cells in the process of neural cell differentiation (FIG. 4).

### Example 4. Preparation of PLGF expression recombinant-AAV (adeno-associated virus) vector

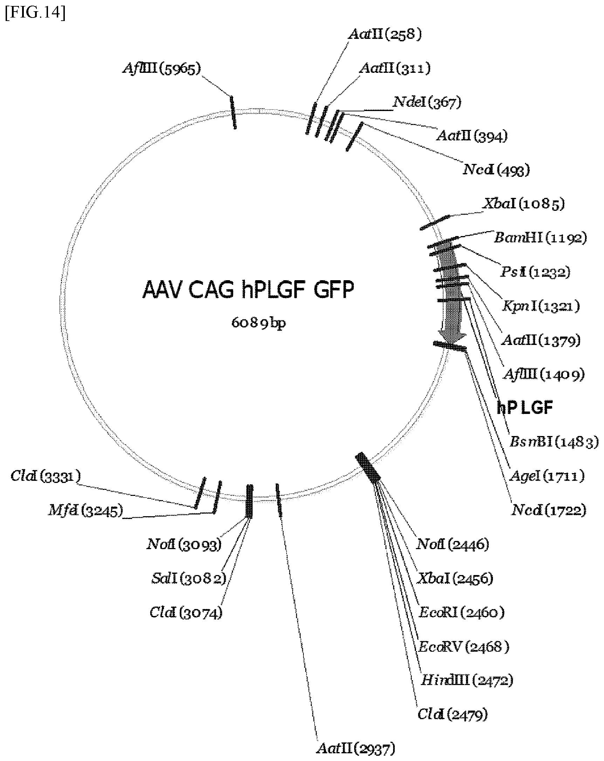

A PLGF gene (SEQ ID NO: 2) was inserted into an adeno-associated virus 2(AAV2) vector to produce a PLGF expression vector (PLGF-AAV, FIG. 14) (SEQ ID NO: 7), which was prepared as a recombinant virus and amplified, concentrated to 10¹²⁻¹³ vg/ml. Then, human mesenchymal stem cells (hMSC) were treated at an MOI of 10⁴. As the human mesenchymal stem cells, a bone marrow-derived MSC cell line prepared by Catholic University of Korea was used, which was cultured using 10% FBS medium or MSC GM (Cambrix) medium.

In addition, as a control, recombinant GFP-AAV containing a GFP expression vector (GFP-AAV) in which the GFP gene (SEQ ID NO: 8) was inserted into the AAV2 vector was prepared as a control virus, and concentrated to 10¹²⁻¹³ vg/ml, and then MSC was treated with MOI 10⁴.

The hMSC treated with the PLGF-AAV or control GFP-AAV was treated with trypsin before injection into the brain, separated from the culture dish, and then treated with a trypsin inhibitor (300 mg/60ml N2 media). After accurately counting the number of live cells using trypan blue, it was adjusted to 7.5×10⁴/µl to be suspended in an injection medium (0.7%p/s, 20mM HEPES (pH7.2), 0.5% glucose in saline), and 2 ul was injected into the brain.

### Example 5. Preparation of a memory impairment and autism model induced by ibotenic acid (IBO)

66-week-old male Sprague-Dawley rats (200-250 g) were purchased from the Orient Animal Breeding Center (Charles River Research Institute, Gyeonggi-do, Korea). The rats were randomly housed 2-3 rats per cage to avoid social stress. The breeding condition was a condition in which a certain temperature range (23 ± 2°C), humidity range (60 ± 10%) and a 12-hour light-dark cycle were maintained and free access to water and feed was provided.

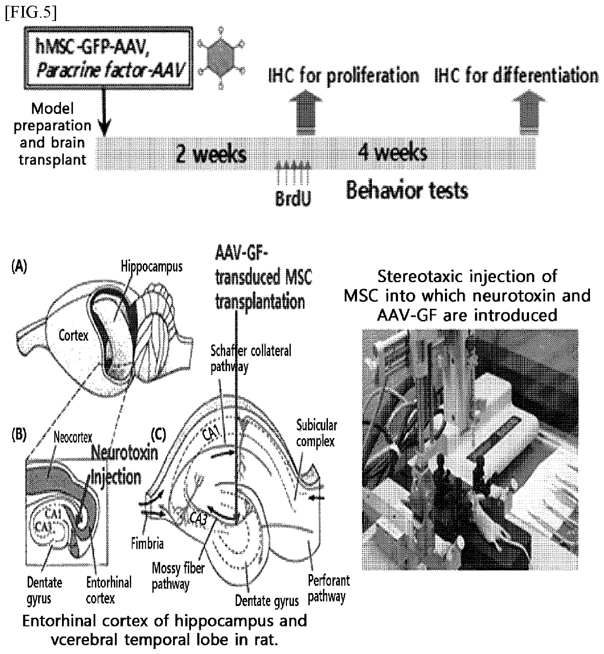

Memory impairment and autism models were prepared by administering IBO to the entorhinal cortex of the rats, wherein the IBO destroys granule cells of the dentate gyrus (DG) and pyramidal cells of the hippocampus, which receive nerve signals input from the entorhinal cortex. Specifically, the rats were anesthetized with equitensin (10% ethanol containing 350 mM pentobarbital sodium, 250 mM chloral hydrate, 85 mM MgSO and 40% propylene glycol). The incisor bar of a stereotaxic device (Stoelting Co., U.S.A.) was placed 3.4 mm below the interaural line, and the injection needle angle was fixed at 10 positions right from the center of the sagittal plane. 1.5 ul of IBO (1 mg/ml) was injected into the entorhinal cortex of the rat using the stereotaxic device. The IBO was injected into three locations of first, AP: -8.4, ML: -4.8, DV: -4.6; Second, AP; -8.4, ML: -4.8, DV: 2.3; Third, AP: -8.8, ML: - 3.65, DV: -3.4 (FIG. 5).

After 2-3 IBO injections, each group was randomly classified into an IBO-administrated group (negative control group, IBO), and a GFP-AAV-hMSC-administrated group (GFP-AAV) and a PLGF-AAV-hMSC-administrated group (PLGF-AAV) as a control group. A rest period of one week was provided. No drug was administered to the Con group (Control, positive control group) consisting of normal rats; saline was administered to the IBO-administrated group; MSC introduced with GFP-AAV by the method of Example 4 was administered to the GFP-AAV-hMSC-administrated group (GFP-AAV); and MSC introduced with PLGF-AAV by the method of Example 4 was administered to the PLGF-AAV-administrated group (PLGF-AAV).

### Example 6. Investigation of the effects of enhancing memory ability according to administration of PLGF

The effects of enhancing memory ability according to administration of PLGF-AAV-hMSC was investigated for the IBO-administered memory impairment and autism model prepared as in Example 5.

### 6-1. Passive avoidance test

Passive avoidance test was performed as described in Heo Het al. (J Ethnopharmacol, 2009) (13 weeks old rat, Con group n=5, IBO-administrated group n=4, GFP-AAV-administrated group n=6, PLGF-AAV-administrated group n=6). This test is composed of a semi-automatic system with a shuttle room. These rats were trained to escape the light by entering a dark room through an acrylic door when the light was turned on. This training was repeated 3 times a day for 3-4 days until the rat entered the dark room within 20 seconds (adaptation). All rats were successfully trained to enter the dark room within 20 seconds on the last training day. After performing the adaptation training on the last day of training, the rats were placed in a bright room, and when they entered the dark room, the door was closed by hand, and an electric shock (1 mA, based on 300 g) was applied to the paws through a grill for 3 seconds. Exactly 24 hours after the adaptation training, the rats were placed in the bright room again, and the latency time to enter the dark room was measured for 720 seconds (confinement experiment).

### 6-2. Moris water maze experiment

The same number of rats as in each group of Example 6-1 was subjected to the Morris water maze experiment in a round, rust-free and inner surface-white swimming pool (diameter 160 cm; height 60 cm). The pool was filled with water (maintained at 23.0 ± 1.0°C) to a depth of 50 cm, and an invisible platform (15 cm, round and white) was hidden 1.0 cm below the water surface and placed in the center of the northeast quadrant. Each rat was trained once a day for 4 days to find the hidden platform. Training was started by randomly placing the rats in the water so as to the pool wall in one of the four quadrants. During each training, the rats were allowed to find the platform for up to 60 seconds and to rest for 1 minute after finding the platform. The average time of the four quadrant tests was defined as the waiting time for escape per group on the training day. The final experiment measured the time the rat stayed where the platform was for 60 seconds after removing the platform (probe trial). Swimming time and training length were tracked and recorded using a video camera. The tracking was performed along the trajectory of the rat indicated by black dots on a white background. The captured video pictures were analyzed with a video tracking system (Ethovision water maze program, Noldus Information Technology, Wogeningen, The Netherlands). The analyzed information included the time of swimming in the target quadrant, and the number of crossings the imaginary platform to find the removed platform.

As a result of the experiment of Examples 6-1 to 6-3, as shown in FIGS. 6A-B, the animals in which hMSC into which PLGF-AAV was introduced (PLGF-AAV) was transplanted showed memory improvement in all of the passive avoidance test and Morris water maze test for measuring memory ability.

### Example 7. Investigation of the recovery of social behavior in a social open field according to the administration of PLGF

The defects in sociability of the IBO-administered memory impairment and autism model prepared as in Example 5, and whether these defects could be recovered when PLGF-AAV-hMSC was administered were measured by behavioral experiments.

Specifically, the sociability was tested in the social open field. Rats were adapted to the recording environment for 30 min, and then adapted to a social open field space composed of black acrylic for 10 min. Strange rats were placed in a cylindrical cage allowing for olfaction and minimal contact, and placed in the center of the edge of the arena. The movements of the test rats were recorded for 10 minutes and analyzed by the ethovision 3.1 program. In addition, the range of sociability was analyzed by setting 10 cm around the cylinder in which the strange rats were placed.

As a result, the time spent in the destination area and the time showing interest in the target individual (sniffling) were similarly shorter than those of normal rats in the IBO-administered group and the GFP-AAV-hMSC-administered group (GFP-AAV), but were significantly longer in the PLGF-AAV-hMSC-administered group (PLGF-AAV) compared to the IBO-administered group or the GFP-AAV group (FIG. 7), showing that PLGF administration restored social behavior.

### Example 8. Recovery of social behavior in 3 rooms according to the administration of PLGF

A three-room experiment was conducted to see if social cognition, sociability and social sexual preference were restored when PLGF-AAV-hMSC was administered to the IBO-administered memory impairment and autism model prepared as in Example 5.

Specifically, an experimental site is composed of three rooms having a transparent acrylic wall and a small door, wherein each room was 50 cm long x 100 cm wide x 50 cm high. The olfaction and minimal contact of the test rats to the strange rats was allowed through the cylindrical cage. Prior to the test, the test rats were placed in the middle room, and allowed to adapt for 5 minutes after both doors were closed. The test was divided into the following sessions I, II and III and performed.

### <Session I> None vs Stranger

To measure sociability, two cylindrical cages were placed in each edge room, one containing a strange rat and the other empty. The test rats were placed in the middle room, moved freely in the three rooms, and had access to two cages for 10 minutes. The movement of the rat was recorded and analyzed as the areas of none and strange rat 1 through ethovision 3.1 program.

### <Session II> Familiar vs Stranger

In order to build a new sociality for a new object and analyze the social cognition of the object that has already formed sociality, a new rat (strange rat) were added to the cage that was new in session I. After opening both doors so that the test rat can access all three chambers, the test rat was placed in the middle chamber and allowed to move freely for 10 minutes. The movement of the rat was recorded and analyzed as the areas of a familiar rat and a strange rat through ethovision 3.1 program.

### <Session III> Sexual Preference

To measure sexual preference and social cognition by analyzing contact with female rats, female and male rats were placed in cylindrical cages in each room, and the cages in which the test rats were in contact more frequently, and the contact times were measured. The movement of the rat was recorded and analyzed as access time and frequency for each cage per session through ethovision 3.1 program.

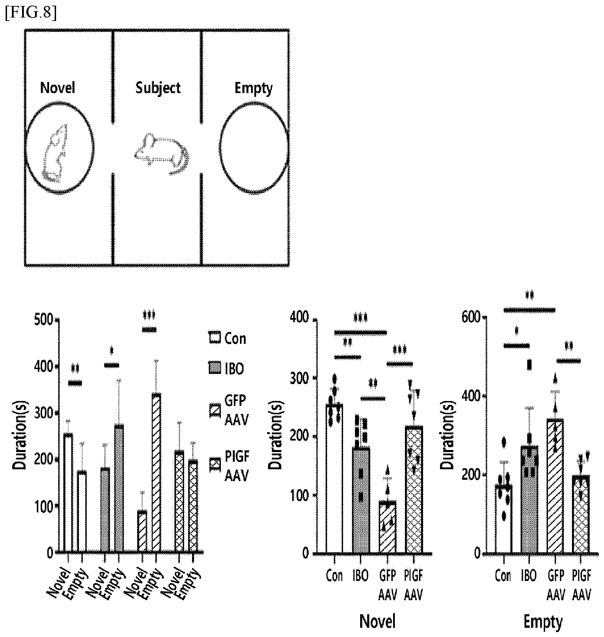

As shown in FIG. 8, in session I, the time spent in each area was analyzed by dividing it into an empty area and an area containing strange rats, and as a result, the time spent in the destination area was significantly reduced in the IBO-administered group compared to the normal group, and was shorter in the GFP-AAV-hMSC-administered group than in the IBO-administered group, but tended to be significantly longer in the PLGF-AAV-hMSC-administered group than in the IBO-administered group, although it was not significant, and was significantly different compared to the GFP-AAV-HMSC-administered group. On the other hand, the time spent in the empty area without the target rats was the longest in the GFP-AAV-hMSC-administered group, and in the PLGF-AAV-hMSC-administered group, it was shortened to a level similar to that of the normal group.

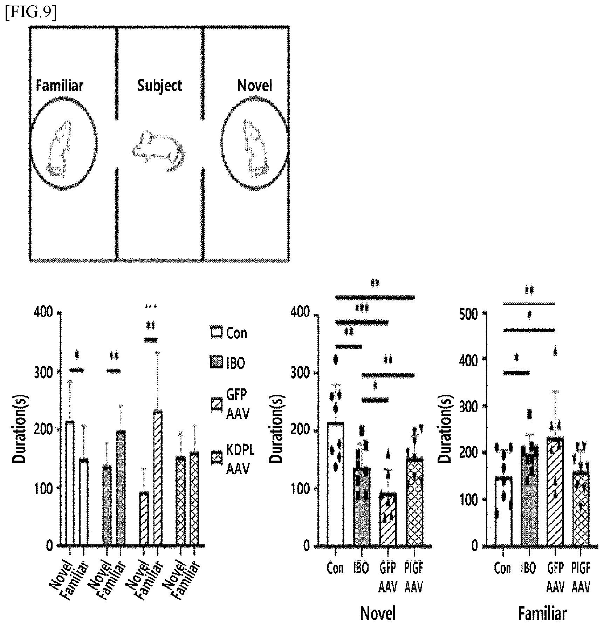

As shown in FIG. 9, in Session II, how well the rats had already formed sociability was investigated by recognizing a familiar rat in one room and a strange rat in another room and forming sociability with a new object. As a result, the time spent in the strange rat area was significantly decreased in the IBO-administered group compared to the normal group, and was shorter in the GFP-AAV-hMSC-administered group than in the IBO-administered group. However, the time in the PLGF-AAV-hMSC-administered group tended to be longer than that in the IBO-administered group, but was not significant, and was significantly longer than in the GFP-AAV-hMSC-administered group. On the other hand, the time spent in the familiar rat area was the longest in the GFP-AAV-hMSC-administered group, and in the PLGF-AAV-hMSC-administered group, it was shortened to a level similar to that of the normal group.

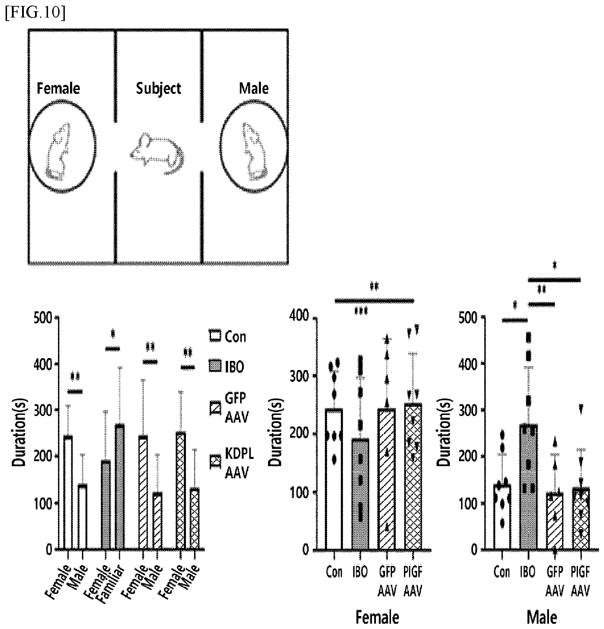

In addition, as shown in FIG. 10, as a result of confirming the social sex preference in Session III, the time spent in the female rat area tended to decrease in the IBO-administered group, but in both the GFP-AAV-hMSC-administered group and the PLGF-AAV-hMSC-administered group, it was recovered to a level similar to that of the normal group. On the other hand, the time spent in the male rat area was similarly short in all of the Con group, the GFP-AAV-hMSC-administered group and the PLGF-AAV-hMSC-administered group, except for in the IBO-administered group.

From this, it can be seen that the administration of PLGF restores social cognition, sociability and social sexual preference.

### Example 9. Immunological tissue staining experiment

For immunohistochemical analysis after the behavioral experiment of Example 8, brain tissues of 4 or more rats from each group were obtained and brain sections were immunostained.

### 9-1. Immunohistochemical analysis - TUNEL

As an experimental method, the one described in Heo et al. (2009) was modified and used. Rats were transcardially perfused with 4% PFA in PBS, and immersed and fixed in 4% PFA in PBS for 4 hours. Thereafter, the brain tissue was non-frozen in 30% sucrose in PBS, then frozen to optimal cutting temperature (OCT) mixture and stored at -80°C. Brain tissue sections were cut in a cold place through a 35 µm-thick coronal plane. The brain tissue sections were immersed in a stock solution (30% glycerol, 30% ethylene glycol in PBS) and stored at 4°C.

Thereafter, a TUNEL assay was performed to analyze the death degree of brain neural cells. The stored brain slices were fixed at room temperature for 10 minutes using 4% paraformaldehyde, washed twice with PBS, and were enzymatically reacted for 1 hour in a 37°C thermostat in which humidity was maintained according to the protocol of the TUNEL assay kit (Roche, 11684795910). Thereafter, they were washed three times with PBS, and the cell nuclei were labeled with 1 ug/ml propidium iodide (Sigma, P4864, 1:3000) to analyze cell death.

As a result, as shown in FIG. 11, cell death in the IBO-administered group was significantly increased by two or more times, but the cell death was significantly suppressed in the PLGF-AAV-hMSC-administered group compared to the IBO-administered group and the GFP-AAV-administered group and recovered to a degree similar to the normal group, showing that the PLGF inhibited death of brain nerve cells and increased survival in the memory impairment and autism model.

### 9-2. Immunohistochemical analysis - BrdU, SOX2, NeuN, CNPase, vGluT1, GAD67, ChAT

The brain tissue stored in the same method as the immunostaining of Example 9-1 was washed twice with PBS, permeated in 0.5% Triton X-I00 for 20 minutes, and cultured in 2N HCl at 37°C for 30 minutes. Thereafter, blocking was performed in 15% standard serum, 3% bovine serum albumin (bio-WORLD, Dublin, OH, USA) and 0.1% Triton X-100 in a free-floating state for 2 hours. The tissue was doubly stained at 4°C for 16 hours using BrdU (abcam, 1:700) antibody, SOX2 (R&D system, MAB2018, 1:2000), NeuN (Millipore, MAB377B, 1:700), CNPase (Abcam, ab6319, 1:700), vGluT1 (Millipore, MAB5502, 1:700), GAD67 (Millipore, MAB5406, 1:2000), and ChAT(Millipore, AB144p, 1:200) as primary antibodies. As secondary antibodies, mo-Alexa 488 (Invitrogen, A21202, 1:700), mo-Cy3 (Jackson lab, 715-165-151, 1:500), rb-Alexa 488 (Jackson lab, 711-546-152, 1:700), and rat-Alexa 488 (Abcam, ab6326, 1:700) were used. Immunostained tissues were scanned with a confocal microscope (LSM510, LSM800 Carl Zeiss, Oberkochen, Germany).

The number of differentiation markers in the hippocampus of each experimental animal was shown by counting the number of each cells labeled by fluorescently labeling each differentiation marker in the photographed slides. Here was counted the number of cells labeled with the secondary antibody that is precisely matched in shape and location and has the shape of a cell.

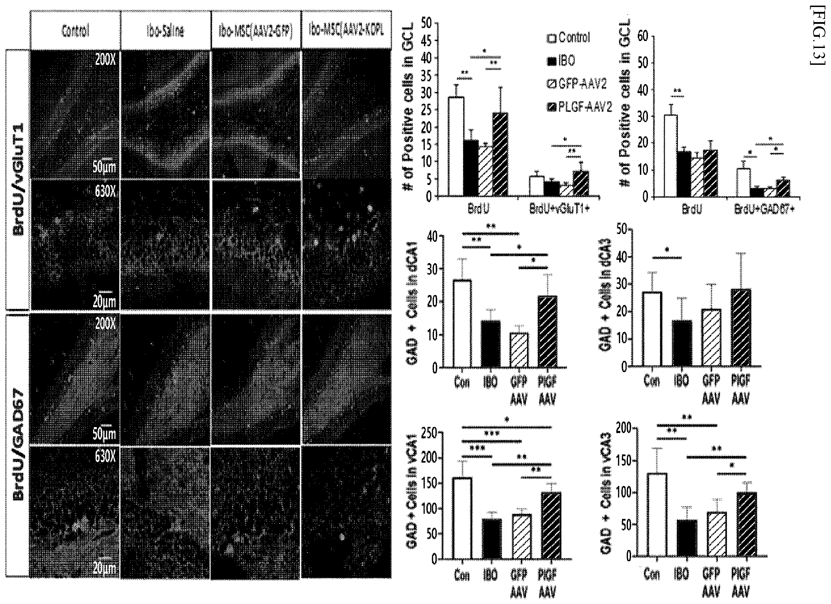

As a result, as shown in FIGS. 12 and 13, when cells that were proliferating at that time were labeled and stained with BrdU, the number of cells expressing SOX2, a marker of neural stem cells, decreased in the IBO-administered group, increased in the GFP-AAV2-hMSC-administered group, and showed a tendency to increase more in the PLGF-AAV-administered group than in the normal group. After 4 weeks, at the time of the differentiation of neural cells, NeuN-expressing cells, a marker of mature neural cells, decreased significantly in the IBO group and the GFP-AAV2-hMSC-administered group than in the normal group, and significantly increased in the PLGF-AAV-hMSC-administrated group than in the above two groups. The number of oligodendrocytes expressing CNPase also increased compared to the IBO-administered group or the GFP-AAV-administered group. Glutamate neural cells (expressing vGluT1) and GABAergic neural cells (expressing GAD) also increased compared to the IBO-administered group and the GFP-AAV-hMSC-administered group. The GABAergic neural cells also increased in CA1 of the dorsal hippocampus, which is known to be associated with memory ability, but also significantly increased in CA1 and CA3 areas of the ventral hippocampus, known to be related to sociality (Torcato Meira et al., Nature communications, 2018) by the administration of PLGF-AAV-hMSC compared to the IBO group and the GFP-AAV-hMSC-administrated group. In particular, in the CA3 area, the increase in the dorsal area was not significant, but a significant increase was shown in the ventral area where the area was much larger.

According to the results of the above examples, the administration of PLGF promotes the proliferation and differentiation of neural stem cells in memory impairment and autism models to induce nerve regeneration and enhance memory ability, and in particular, promotes the differentiation of GABAergic nerve cells in the dorsal hippocampus to ameliorate decline in social cognition, sociability and social sexual preference, whereby a composition for preventing or treating neuropsychologic diseases such as autism, ADHD, mental retardation disorder, and developmental disorder can be provided.

From the above description, those skilled in the art to which the present invention pertains will understand that the present invention may be embodied in other specific forms without changing the technical spirit or essential characteristics thereof. In this regard, it should be understood that the embodiments described above are illustrative in all respects and not restrictive. The scope of the present invention should be construed as including the meaning and scope of the claims to be described later rather than the detailed description above, and all changed or modified forms derived from the equivalent concepts thereof.

## Claims

1. A pharmaceutical composition for preventing or treating neuropsychologic diseases, the composition comprising, as an active ingredient, (a) placental growth factor (PLGF); or (b) a peptide derived therefrom, or a fragment thereof.

2. The pharmaceutical composition according to claim 1, wherein the PLGF comprises any one or more selected from amino acid sequences of SEQ ID NOs: 1 to 3 or amino acid sequences having 80% or more sequence identity therewith.

3. The pharmaceutical composition according to claim 1, wherein the neuropsychologic diseases are any one or more selected from the group consisting of autism, ADHD, mental retardation disorder and developmental disorder.

4. The pharmaceutical composition according to claim 1, wherein (a) PLGF or (b) a PLGF-derived peptide or fragment thereof is included in any one form selected from the group consisting of a polypeptide sequence of PLGF or a fragment thereof; a polynucleotide sequence encoding the polypeptide sequence or a fragment thereof; and a vector comprising the polynucleotide sequence.

5. The pharmaceutical composition according to claim 1, wherein (a) PLGF or (b) a PLGF-derived peptide or fragment thereof promotes the regeneration of nerve cells in a hippocampus.

6. The pharmaceutical composition according to claim 1, wherein (a) PLGF or (b) a PLGF-derived peptide or fragment thereof promotes the differentiation of nerve cells in a hippocampus.

7. The pharmaceutical composition according to claim 1, wherein (a) PLGF or (b) a PLGF-derived peptide or fragment thereof enhances memory.

8. The pharmaceutical composition according to claim 1, wherein (a) PLGF or (b) a PLGF-derived peptide or fragment thereof enhances sociability or social cognitive ability.

9. A method for preventing or treating neuropsychologic diseases, comprising administering the composition of any one of claims 1 to 8 to an individual, excluding humans.

10. A health functional food composition for preventing or ameliorating neuropsychologic diseases, the composition comprising, as an active ingredient, (a) PLGF; or (b) a peptide derived therefrom, or a fragment thereof.

11. A feed composition for preventing or ameliorating neuropsychologic diseases, the composition comprising, as an active ingredient, (a) PLGF; or (b) a peptide derived therefrom, or a fragment thereof.
